# EUROPEAN PATENT APPLICATION

(11) **EP 4 389 188 A1**
(43) Date of publication of application: **26.06.2024**
(21) Application number: 22858258.1
(22) Date of filing: 25.07.2022
(51) Int. Cl.: A61M 25/10, A61M 25/00

(54) **BALLOON FOR CATHETER AND BALLOON CATHETER**

(30) Priority: 16.08.2021 JP 2021132155
(71) Applicant: Goodman Co., Ltd., Aichi 460-0008 (JP)
(72) Inventor: OKAMOTO, Mitsumasa, Seto-shi, Aichi 489-0976 (JP); YOSHINAGA, Shizuya, Seto-shi, Aichi 489-0976 (JP); KONDO, Shoma, Seto-shi, Aichi 489-0976 (JP); YAMAMOTO, Shuhei, Seto-shi, Aichi 489-0976 (JP); NAKAMURA, Yuta, Seto-shi, Aichi 489-0976 (JP)
(74) Representative: Epping - Hermann - Fischer
(86) International application number: PCT/JP2022/028566
(87) International publication number: WO 2023/021936

(57) **Abstract**

A balloon catheter (1A) includes a balloon (3) extending in an extending direction, and an element (4A) disposed at at least a part of an outer surface of the balloon (3). The element includes linear members (41, 42) extending in the extending direction between a second distal end portion (40D) and a second proximal end portion (40P). The linear members (41, 42) include a plurality of slits (51, 52) arranged in the extending direction. The linear members (41, 42) include a dense region (4M) where an interval between each of the plurality of slits (51, 52) is smaller than a predetermined first threshold value, and a sparse region (4S) where the interval between each of the plurality of slits is larger than a second threshold value being a value equal to or greater than the first threshold value.

## Description

### Technical Field

The present invention relates to a balloon for a catheter and a balloon catheter.

### Background art

Patent Literature 1 discloses a balloon catheter including a linear portion provided with a cutout portion. The balloon catheter bends easily originating at the cutout portion. Thus, when a balloon is passed through a curved blood vessel, it is possible to suppress a deterioration in trackability of the balloon with respect to the blood vessel as a result of the linear portion being held in a fully extended state.

### Citation List

### Patent Literature

Patent Literature 1: WO2020/255923A1

### Summary of Invention

It is possible for a user of the above-described balloon catheter to deploy characteristics of being able to form a bending behavior at the linear portion, to thus apply a folding behavior in the balloon before use, and to use the balloon catheter for treatment in this state. In particular, this case is effective since it is possible to achieve excellent passability when passing the balloon inside a blood vessel that is curved in a specific shape. However, forming the bending behavior in the balloon in an appropriate shape corresponding to the blood vessel is sometimes difficult even for a proficient user. There is thus a problem that the excellent passability can sometimes not be achieved inside the blood vessel, as the shape of the balloon is not stable due to variations in the method of forming the folding behavior.

An object of the present invention is to provide a balloon for a catheter and a balloon catheter capable of forming a bending behavior in a stable manner into a shape desired by a user.

A balloon for a catheter according to a first aspect of the present invention includes a balloon and an element. The balloon extends between a first distal end portion being an end portion on one side in an extending direction and a first proximal end portion being an end portion on another side. The element is disposed at at least a part of an outer surface of the balloon. The element includes at least one linear member extending in the extending direction between a second distal end portion being an end portion close to the first distal end portion and a second proximal end portion being an end portion close to the first proximal end portion. The linear member includes a plurality of slits arranged in the extending direction. The linear member includes a dense region where an interval between each of the plurality of slits is smaller than a predetermined first threshold value, and a sparse region where the interval between each of the plurality of slits is larger than a second threshold value being a value equal to or greater than the first threshold value.

According to the first aspect, the linear member easily bends at the dense region where the interval between each of the plurality of slits is relatively smaller. Thus, the bending behavior of bending at the position of the dense region is easily formed in the balloon. As a result, a user of the balloon for a catheter can stably apply the bending behavior to the balloon to become a desired shape.

In the balloon for the catheter according to the first aspect, the element may include a plurality of the linear members. The plurality of linear members may be arranged in a circumferential direction centered on a center axis of the balloon. In this case, the bending behavior is even more easily formed in the balloon using the plurality of linear members. Thus, compared to a case in which only the single linear member is provided, the balloon for a catheter can stably maintain the balloon in the state in which the bending behavior is applied.

In the balloon for the catheter according to the first aspect, the plurality of linear members may include at least a first linear member and a second linear member. The dense region of the first linear member and the dense region of the second linear member may be disposed at different positions from each other in the extending direction. By causing the positions of the dense regions of the first linear member and the second linear member to be different from each other, the balloon for a catheter can dispose the dense region on the inner side, where the curvature is larger when the balloon is in the bent state, and can dispose the sparse region on the outer side where the curvature is smaller. Note that the dense region of the linear member bends easily with the larger curvature and the sparse region of the linear member bends easily with the smaller curvature. Thus, the balloon for a catheter can stably maintain the balloon in the state in which the bending behavior is applied.

In the balloon for the catheter according to the first aspect, the first linear member may include a first dense region and a second dense region as the dense region. The second linear member may include a third dense region as the dense region. The first dense region, the second dense region, and the third dense region may be disposed at different positions from each other in the extending direction. In the extending direction, the third dense region may be disposed between the first dense region and the second dense region. In this case, in the balloon for a catheter, a bending behavior can be easily formed, in the balloon, of bending alternately in directions intersecting the extending direction.

In the balloon for the catheter according to the first aspect, the first linear member may include a first dense region as the dense region. The second linear member may include a second dense region as the dense region. The first dense region and the second dense region may be disposed at different positions from each other in the extending direction. In the extending direction, the first dense region and the second dense region may be disposed between the first distal end portion and a center position between the first distal end portion and the first proximal end portion of the balloon. In this case, in the balloon for a catheter, a bending behavior can be easily formed in which the curvature is larger at the distal end portion of the balloon.

In the balloon for the catheter according to the first aspect, the plurality of linear members may include at least a first linear member and a second linear member. The first linear member may include a first dense region as the dense region. The second linear member may include a second dense region as the dense region. The first dense region and the second dense region may be disposed at the same position as each other in the extending direction. In the extending direction, the first dense region and the second dense region may be disposed between the first distal end portion and a center position between the first distal end portion and the first proximal end portion of the balloon. In this case, in the balloon for a catheter, the bending behavior can be easily formed in which the curvature is larger at the distal end portion of the balloon.

In the balloon for the catheter according to the first aspect, the plurality of linear members may include at least a first linear member and a second linear member. A hardness at the dense region of the first linear member may be different from a hardness at the dense region of the second linear member. In this case, the balloon for a catheter can dispose the linear member having the relatively soft dense region on the inner side where the curvature is larger and can dispose the linear member having the relatively hard dense region on the outer side where the curvature is smaller when the balloon is in the bent state. Note that in the linear member, the softer portion bends easily with the larger curvature and the harder portion bends easily with the smaller curvature. Thus, the balloon for a catheter can stably maintain the balloon in the state in which the bending behavior is applied.

In the balloon for the catheter according to the first aspect, in the extending direction, a position at which the dense region of the linear member is disposed may include a center position in the extending direction between the first distal end portion and the first proximal end portion of the balloon. In this case, the user can easily form the bending behavior of the balloon in which the curvature is larger at the center position in the extending direction of the balloon.

In the balloon for the catheter according to the first aspect, the balloon may include an inflatable portion, a distal end connecting portion, and a proximal end connecting portion. The inflatable portion may have a tube shape and extending in the extending direction. The distal end connecting portion may be a portion extending to the first distal end portion from an end portion on the one side in the extending direction of the inflatable portion. A diameter of an end portion connected to the inflatable portion may be larger than a diameter of the first distal end portion. The proximal end connecting portion may be a portion extending to the first proximal end portion from an end portion on the other side in the extending direction of the inflatable portion. A diameter of an end portion connected to the inflatable portion may be larger than a diameter of the first proximal end portion. In the extending direction, a position at which the dense region of the linear member is disposed may include the center position and include two equal division positions dividing the inflatable portion into thirds in the extending direction. In this case, the user can suppress the balloon from bending at an acute angle when forming the bending behavior in which the curvature is larger at the center position in the extending direction of the balloon.

In the balloon for the catheter according to the first aspect, a greatest depth, in a radial direction centered on a center axis of the balloon, of each of the plurality of slits may become gradually deeper toward a central portion of the dense region from both end portions thereof in the extending direction. The deeper the depth of the slits in the linear member, the more it is possible to bend with the larger curvature. In other words, the dense region of the linear member can bend with the larger curvature the closer to the central portion. When the linear member bends at the dense region, the closer to the central portion of the dense region, the more easily the curvature becomes larger. Thus, the user can form the bending behavior in the balloon by easily causing the linear member to be in the bent state.

In the balloon for the catheter according to the first aspect, each of the plurality of slits may be formed by a pair of side surfaces facing each other in the extending direction. A greatest width, in the extending direction, between the pair of side surfaces of each of the plurality of slits may become gradually larger toward a central portion of the dense region from both end portions thereof in the extending direction. The larger the maximum width between the pair of side surfaces of the slits in the linear member, the more it is possible to bend with the larger curvature. In other words, the dense region of the linear member can bend with the larger curvature the closer to the central portion. When the linear member bends at the dense region, the closer to the central portion of the dense region, the more easily the curvature becomes larger. Thus, the user can form the bending behavior in the balloon by easily causing the linear member to be in the bent state.

In the balloon for the catheter according to the first aspect, a hardness of the linear member may become gradually softer toward a central portion of the dense region from both end portions thereof in the extending direction. The softer the hardness of the linear member, the more easily the linear member bends and tracks changes in the curvature of a blood vessel. In other words, the dense region of the linear member bends more easily the closer to the central portion. When the linear member bends and tracks the changes in the curvature of the blood vessel, the linear member bends more easily the closer to the central portion. Thus, the balloon for a catheter can easily bend the balloon while tracking changes in the curvature of the blood vessel.

In the balloon for the catheter according to the first aspect, the dense region of the linear member may be covered by a radio-opaque material. In this case, the balloon for a catheter can cause the user to recognize the position of the dense region of the linear member. Thus, during treatment using the balloon for a catheter, the user can easily dispose the dense region of the linear member at a curved location in the blood vessel.

In the balloon for the catheter according to the first aspect, at least the dense region of the linear member may be formed from a radio-opaque material. In this case, the balloon for a catheter can cause the user to recognize the position of the dense region of the linear member. Thus, during treatment using the balloon for a catheter, the user can easily dispose the dense region of the linear member at a treatment location in the blood vessel.

In the balloon for the catheter according to the first aspect, a boundary section between an outer surface of the linear member and the slit has a rounded shape. In this case, the balloon for a catheter can reduce a possibility of damage to the blood vessel as a result of the slit acting on the blood vessel.

In the balloon for the catheter according to the first aspect, the balloon and the element may be formed from the same material. In this case, the balloon including the element can be easily manufactured.

A balloon catheter according to a second aspect of the present invention includes the balloon for a catheter according to the first aspect and a shaft. The shaft may extend between a third distal end portion being an end portion on one side and a third proximal end portion being an end portion on another side. The first distal end portion of the balloon for a catheter may be disposed close to the third distal end portion of the shaft, and the first proximal end portion of the balloon for a catheter may be disposed at a position separated from the third distal end portion of the shaft to the third proximal end portion side. The shaft includes a radio-opaque material indicating a position of the dense region.

According to the second aspect, the balloon for a catheter can cause the user to recognize the position of the dense region of the linear member, in the balloon. Thus, during treatment using the balloon catheter, the user can easily dispose the dense region of the linear member of the balloon at a curved location in the blood vessel.

### Brief description of the drawings

Fig. 1 is a view showing a balloon catheter 1A, a partially enlarged view of a slit 51, and a cross-sectional view as seen in the direction of arrows along a line A-A.
Fig. 2A is a view showing a state in which a bending behavior is formed in the balloon catheter 1A.
Fig. 2B is a view showing a state in which in which the bending behavior is formed in the balloon catheter 1A.
Fig. 2C is a view showing a state in which the bending behavior is formed in the balloon catheter 1A.
Fig. 3 is a view of a balloon catheter 1B.
Fig. 4A is a view showing a state in which a bending behavior is formed in the balloon catheter 1B.
Fig. 4B is a view showing a state in which the bending behavior is formed in the balloon catheter 1B.
Fig. 4C is a view showing a state in which the bending behavior is formed in the balloon catheter 1B.
Fig. 5 is a view showing a balloon catheter 1C.
Fig. 6A is a view showing a state in which a bending behavior is formed in the balloon catheter 1C.
Fig. 6B is a view showing a state in which the bending behavior is formed in the balloon catheter 1C.
Fig. 6C is a view showing a state in which the bending behavior is formed in the balloon catheter 1C.
Fig. 7 is a view showing a balloon catheter 1D.
Fig. 8A is a view showing a state in which a bending behavior is formed in the balloon catheter 1D.
Fig. 8B is a view showing a state in which the bending behavior is formed in the balloon catheter 1D.
Fig. 8C is a view showing a state in which the bending behavior is formed in the balloon catheter 1D.
Fig. 9 is a view showing a balloon catheter 1E.
Fig. 10 is a view showing a balloon catheter 1F.
Fig. 11 is a view showing a balloon catheter 1G.

### Description of Embodiments

Embodiments (balloon catheters 1A to 1G) of a balloon catheter 1 according to the present invention will now be described with reference to the accompanying drawings. The referenced drawings are used to describe technical features that can be employed in the present invention. The described configuration and the like of the device is not intended to be limited thereto and is merely an example for explanation purposes. Using a balloon 3, the balloon catheter 1 can dilate a stenotic lesion formed in a blood vessel, or can cut the lesion using elements 4 (elements 4A to 4G) to be described later.

### First embodiment (Balloon catheter 1A)

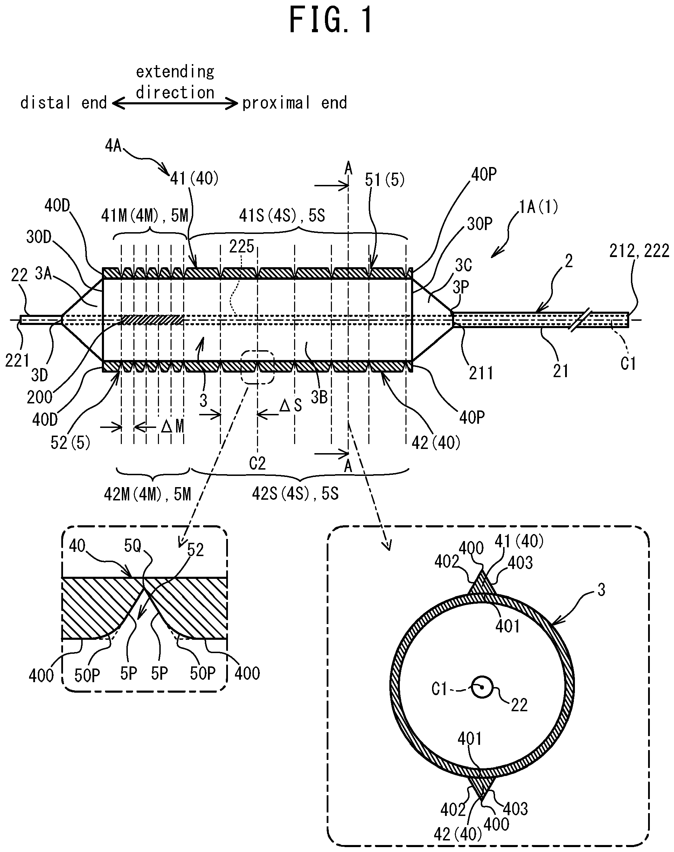

The balloon catheter 1A will be described with reference to Fig. 1. The balloon catheter 1A includes a catheter shaft 2, the balloon 3, and the elements 4A.

### Catheter shaft 2

The catheter shaft 2 has a tube shape. The balloon 3 is connected to an end portion on one side of the tube-shaped catheter shaft 2. A hub that is not shown in the drawings is connected to an end portion on the other side of the catheter shaft 2. The hub can supply compressed fluid to the balloon 3 via the catheter shaft 2.

Hereinafter, the one side from among both ends of the catheter shaft 2 will be referred to as a distal end side. The other side from among both ends of the catheter shaft 2 will be referred to as a proximal end side. The direction extending along the catheter shaft 2 will be referred to as to as an extending direction. An axis passing through the center of the catheter shaft 2 and extending in the extending direction will be referred to as a center axis C1. In a cross section cut on a plane perpendicular to the center axis C1 (hereinafter simply referred to as a cross section), the side closer to the center axis C1 in a radial direction centered on the center axis C1 will be referred to as an inner side, and the side further away from the center axis C1 will be referred to as an outer side.

The catheter shaft 2 has an outer tube 21 and an inner tube 22. The outer tube 21 and the inner tube 22 are both flexible. The inside diameter of the outer tube 21 is larger than the outside diameter of the inner tube 22. Apart from a predetermined portion on the distal end side, the inner tube 22 is disposed inside the lumen of the outer tube 21. The predetermined portion on the distal end side of the inner tube 22 protrudes toward the distal end side from an end (hereinafter referred to as a distal end 211) on the distal end side of the outer tube 21. The end (hereinafter referred to as a distal end 221) on the distal end side of the inner tube 22 is disposed further to the distal end side than the distal end 211 of the outer tube 21. Hereinafter, the predetermined portion on the distal end side of the inner tube 22 will be referred to as a protruding section 225. The end on the proximal end side of the outer tube 21 will be referred to as a proximal end 212. The end on the proximal end side of the inner tube 22 will be referred to as a proximal end 222. The hub is connected to at least the proximal end 212. The material of the outer tube 21 and the inner tube 22 is not particularly limited, and a polyamide resin may be used, for example.

The compressed fluid supplied from the hub passes through a space of the lumen of the outer tube 21 other than the lumen of the inner tube 22. A guide wire that is not shown in the drawings is inserted through the lumen of the inner tube 22.

### Balloon 3

The balloon 3 can change shape between a deflated state and an inflated state, as a result of a change in internal pressure according to whether the compressed fluid is supplied from the hub that is not shown in the drawings. Fig. 1 shows the balloon 3 in the inflated state.

The end (hereinafter referred to as a distal end portion 3D) on the distal end side of the balloon 3 is connected by thermal welding to the inner tube 22, in the vicinity of the distal end 221 of the protruding section 225. Further, the end (hereinafter referred to as a proximal end portion 3P) on the proximal end side of the balloon 3 is connected by thermal welding to the outer tube 21, in the vicinity of the distal end 211. The proximal end portion 3P of the balloon 3 is disposed at a position separated, to the proximal end 222 side, from the distal end 221 of the inner tube 22. The balloon 3 covers the protruding section 225 of the inner tube 22 from the outside. The material of the balloon 3 is not particularly limited, and a polyamide resin may be used, for example.

A distal end connecting portion 3A, an inflatable portion 3B, and a proximal end connecting portion 3C are defined in the balloon 3. The distal end connecting portion 3A is a region extending while increasing in diameter from the distal end portion 3D toward the proximal end portion 3P of the balloon 3 in the inflated state. The proximal end connecting portion 3C is a region extending while increasing in diameter from the proximal end portion 3P toward the distal end portion 3D of the balloon 3 in the inflated state. The inflatable portion 3B is a region sandwiched between the distal end connecting portion 3A and the proximal end connecting portion 3C of the balloon 3 in the inflated state, and the diameter thereof is substantially the same along the extending direction. In the inflated state, the inflatable portion 3B has a tubular shape extending in the extending direction. An end on the distal end side of the inflatable portion 3B will be referred to as a distal end portion 30D, and an end on the proximal end side of the inflatable portion 3B will be referred to as a proximal end portion 30P.

The distal end connecting portion 3A extends to the distal end side toward the distal end portion 3D, from an end connected to the distal end portion 30D of the inflatable portion 3B. The diameter of a cross section of the distal end connecting portion 3A is largest at the end connected to the distal end portion 30D of the inflatable portion 3B, and is smallest at the distal end portion 3D. The proximal end connecting portion 3C extends to the proximal end side toward the proximal end portion 3P, from an end connected to the proximal end portion 30P of the inflatable portion 3B. The diameter of a cross section of the proximal end connecting portion 3C is largest at the end connected to the proximal end portion 30P of the inflatable portion 3B, and is smallest at the proximal end portion 3P.

A center position between the distal end portion 3D and the proximal end portion 3P of the balloon 3 in the extending direction will be referred to as a center position C2. Note that a center position between the distal end portion 30D and the proximal end portion 30P of the inflatable portion 3B of the balloon 3 in the extending direction is aligned with the center position C2.

### Elements 4A

The elements 4A are disposed at the outer surface of the inflatable portion 3B of the balloon 3. The elements 4A are formed from a polyamide resin, and are formed from the same material as the balloon 3. On the other hand, since a state of molecular orientation is different from that of the balloon 3, the hardness of the elements 4Ais harder than that of the balloon 3. The elements 4A include linear members 41 and 42.

The linear members 41 and 42 are provided along the outer surface of the inflatable portion 3B, and face each other on either side of the center axis C1. The shape of each of the linear members 41 and 42 is a triangular column extending along the extending direction. The linear members 41 and 42 are arranged in the circumferential direction centered on the center axis C1. Each of the linear members 41 and 42 extends between the distal end portion 30D and the proximal end portion 30P of the inflatable portion 3B. Hereinafter, when no distinction is made between the linear members 41 and 42, they will be collectively referred to as a linear member 40, or linear members 40. Of the linear member 40, an end portion on the side in the vicinity of the distal end portion 30D of the inflatable portion 3B will be referred to as a distal end portion 40D. Of the linear member 40, an end portion on the side in the vicinity of the proximal end portion 30P of the inflatable portion 3B will be referred to as a proximal end portion 40P.

A cross-sectional shape of the linear member 40 is a triangular shape having a side surface 401 as the base. Of the linear member 40, a point at which side surfaces 402 and 403 other than the side surface 401 intersect each other is an apex 400. A direction from the side surface 401 toward the apex 400 extends to the outer side.

The linear member 40 includes a plurality of slits 5 arranged in the extending direction. The positions in the extending direction of each of a plurality of slits 51 provided in the linear member 41 are the same as the positions in the extending direction of each of a plurality of slits 52 provided in the linear member 42.

Each of the plurality of slits 5 is formed by a pair of side surface 5P facing each other in the extending direction. The interval in the extending direction between the pair of side surface 5P becomes smaller the deeper the depth of each of the plurality of slits 5. Sections at which each of the pair of side surfaces 5P are connected to the apex 400 of the linear member 40, that is, boundary sections 50P in the vicinity of the apex 400, of the boundaries between the side surfaces 402 and 403 and each of the plurality of slits 5, have a rounded shape.

In the radial direction centered on the center axis C1, a length from the apex 400 of the linear member 40 to a deepest portion 5Q at which the pair of side surface 5P intersect each other will be referred to as a greatest depth of each of the plurality of slits 5. The greatest depth is the same for all of the plurality of slits 51 and 52, respectively. A width in the extending direction between the boundary section 50P in the vicinity of the apex 400 of the side surface 402 and the boundary section 50P in the vicinity of the apex 400 of the side surface 403 will be referred to as a greatest width of each of the plurality of slits 5. The greatest width is the same for all of the plurality of slits 51 and 52, respectively.

A distance, in the extending direction, between the respective deepest portions 5Q of two of the slits 5, of the plurality of slits 5, that are adjacent to each other in the extending direction corresponds to an interval between the two slits 5. The intervals between each of the plurality of slits 5 are not uniform. More specifically, the plurality of slits 5 include a dense slit group 5M between each of which the interval is ΔM, and a sparse slit group 5S between each of which the interval is ΔS. The interval ΔM is smaller than a predetermined first threshold value Th1 (ΔM < Th1). The interval ΔS is larger than a predetermined second threshold value Th2 (Th2 < ΔS) and the second threshold value Th2 is equal to or greater than the first threshold value (Th1 ≤ Th2).

Of the linear member 40, a region in which the dense slit group 5M is formed will be referred to as a dense region 4M. Of the linear member 40, a region in which the sparse slit group 5S is formed will be referred to as a sparse region 4S. The dense region 4M of the linear member 41 will be referred to as a dense region 41M, and the sparse region 4S of the linear member 41 will be referred to as a sparse region 41S. The dense region 4M of the linear member 42 will be referred to as a dense region 42M and the sparse region 4S of the linear member 42 will be referred to as a sparse region 42S.

The dense regions 41M and 42M are disposed at the same positions in the extending direction. The dense regions 41M and 42M are disposed closer to the distal end portions 40D of the linear members 40. The sparse regions 41S and 42S are disposed closer to the proximal end portions 40P of the linear members 40. The sparse region 41S is adjacent to the proximal end side of the dense region 41M. The sparse region 42S is adjacent to the proximal end side of the dense region 42M. A length in the extending direction of the sparse regions 41S and 42S is substantially twice a length in the extending direction of the dense regions 41M and 42M.

The dense regions 41M and 42M are disposed to the distal end side of the center position C2. In other words, the dense regions 41M and 42M are disposed between the distal end portion 3D of the balloon 3 and the center position C2 in the extending direction, and more specifically, between the distal end portion 30D of the inflatable portion 3B of the balloon 3 and the center position C2.

The hardnesses of the dense region 41M of the linear member 41 and the dense region 42M of the linear member 42 differ from each other since a state of the molecular orientation differs therebetween. The hardness of the dense region 42M is harder than the hardness of the dense region 41M. On the other hand, the hardnesses of the sparse region 41S of the linear member 41 and the sparse region 42S of the linear member 42 are the same as each other.

Note that a method for causing the hardness to differ between the dense regions 4M and the sparse regions 4S in the linear member 40 is not limited to any particular method. For example, when the balloon 3 and the linear members 40 are formed by blow molding, the hardness of the dense regions 4M may be caused to be harder than the sparse regions 4S as a result of forming the dense regions 4M of the linear members 40 by pressing a high temperature die. Further, for example as a secondary operation after the blow molding, the hardness of the dense regions 4M may be caused to be harder than the sparse regions 4S as a result of heating the dense regions 4M of the linear members 40 at a high temperature.

### Radio-opaque member 200

Of the protruding section 225 of the inner tube 22, a radio-opaque member 200 is provided at a position overlapping the dense regions 4M of the linear members 40 in the extending direction. The radio-opaque member 200 is formed by an alloy of platinum and iridium. The radio-opaque member 200 is fixed to the outer surface of the inner tube 22 by a tubular member formed by the alloy of platinum and iridium being crimped to the protruding section 225 of the inner tube 22. The radio-opaque member 200 does not allow radioactive rays to pass through.

### Operations and effects of first embodiment

When inflatable portion 3B is inflated in a state in which the balloon 3 is disposed at a stenotic location in the blood vessel, the balloon catheter 1A can cause the elements 4A to act on the stenotic location of the blood vessel.

The linear members 40 of the elements 4A bend easily at the dense regions 4M where the interval between each of the plurality of slits 5 is relatively small. Thus, the balloon catheter 1A can easily form a bending behavior in the balloon 3 by bending the balloon 3 at the position of the dense regions 4M. Furthermore, the dense regions 41M and 42M of the linear members 41 and 42 are disposed at the same positions as each other in the extending direction, and are disposed between the distal end portion 30D of the inflatable portion 3B of the balloon 3 and the center position C2. Here, the dense regions 41M and 42M of the linear members 41 and 42 bend more easily than the sparse regions 41S and 42S. Furthermore, portions of the linear members 40 that are soft bend with a larger curvature, and harder portions bend with a smaller curvature. With respect to this, the hardness of the dense region 41M of the linear member 41 and the hardness of the dense region 42M of the linear member 42 differ from each other. The dense region 41M is softer than the dense region 42M.

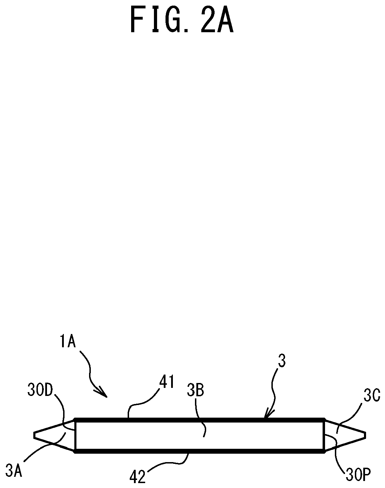

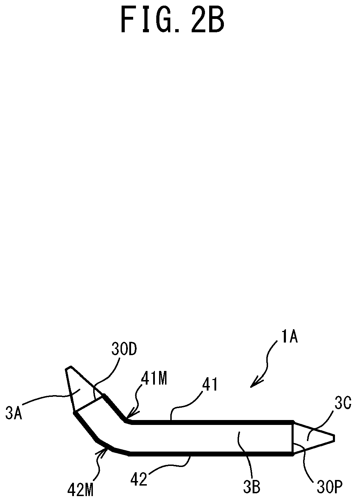

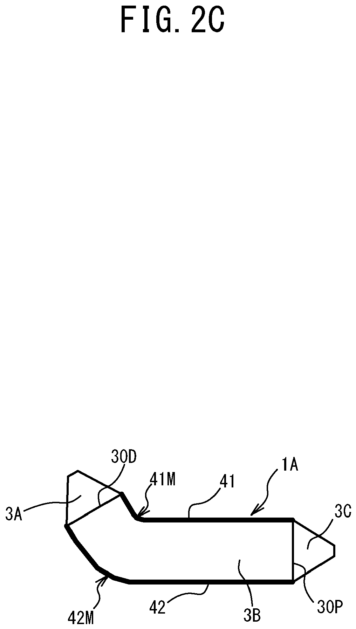

Thus, for example, a user bends the balloon 3 in the deflated state shown in Fig. 2A such that the linear member 41 is disposed to the inner side and the linear member 42 is disposed to the outer side. At this time, the curvature of the dense region 41M is large, and the curvature of the dense region 42M is small. Note that, once bent, the linear members 41 and 42 do not easily return to the original linear shape, and the bent state is maintained. In this way, as shown in Fig. 2B, the user can form the bending behavior in the balloon 3 such that the curvature at a position near the distal end portion 30D of the inflatable portion 3B is large. Note that, the linear members 41 and 42 that have been bent while in the deflated state regulate the shape of the balloon 3 in the inflated state. Thus, as shown in Fig. 2C, the bending behavior applied to the balloon 3 in the deflated state is also maintained even after the balloon 3 is in the inflated state.

The bending behavior is applied to the balloon 3 using the two linear members 40 (the linear members 41 and 42). Thus, the balloon catheter 1A can stably maintain the balloon 3 in the bent state, compared to a case in which only the one linear member 40 is provided.

In the state in which the balloon 3 is bent, the dense region 41M that is relatively softer is disposed on the inner side at which the curvature is larger, and the dense region 42M that is relatively harder is disposed on the outer side at which the curvature is smaller. In this way, the balloon catheter 1A can stably maintain the balloon 3 in the bent state. Furthermore, by causing the hardnesses of the dense region 41M of the linear member 41 and the dense region 42M of the linear member 42 to differ from each other, it is possible to distribute a force applied to the balloon 3 when the user forms the bending behavior. Thus, the user can create the desired bending behavior of the balloon 3, without needing to make delicate adjustments in the force applied to the balloon 3.

The balloon 3 and the elements 4A are formed from the same material. Thus, the balloon 3 including the elements 4A can be easily manufactured. For example, when the balloon 3 is manufactured by blow molding, it is possible to easily manufacture the balloon 3 including the elements 4A by providing grooves for forming the elements 4A in the die.

In the elements 4A, the boundary sections 50P in the vicinity of the apexes 400, of the boundaries between the side surfaces 402 and 403 of the linear members 40 and each of the plurality of slits 5, have the rounded shape. Thus, the balloon catheter 1A can reduce the possibility of each of the plurality of slits 5 acting on the blood vessel and thus causing damage.

The radio-opaque member 200 indicating the position of the dense regions 41M and 42M is provided at the protruding section 225 of the inner tube 22. In this case, the balloon catheter 1A can cause the user to recognize the position of the dense regions 4M of the linear members 40 in the balloon 3. Thus, under X-ray illumination, the user recognizes the position of the dense regions 4M of the linear members 40 using the radio-opaque member 200. As a result, during treatment using the balloon catheter 1A, the user can easily cause the portion of the balloon 3 that bends easily, that is, the dense regions 4M of the linear members 40, to be disposed at a curved section of the blood vessel.

### Special notes relating to first embodiment

The shape of the linear member 40 is not limited to the triangular column, and may be another shape. The apex 400 may be curved. The number of the linear members 40 is not limited to two, and may be one, or may be three or more. When the number of the linear members 40 is three or more, the linear members 40 may be respectively arranged along the outer surface of the inflatable portion 3B at equal intervals in the circumferential direction centered on the center axis C1. The linear member 40 may extend in a helical shape between the distal end portion 40D and the proximal end portion 40P. The linear member 40 need not necessarily extend between the distal end portion 30D and the proximal end portion 30P of the inflatable portion 3B, and may be partially cut.

The sparse regions 4S may be adjacent to the distal end side of the dense regions 4M. The linear member 40 may be provided with at least one of two or more of the dense regions 4M and two or more of the sparse regions 4S. When provided with at least one of two or more of the dense regions 4M and two or more of the sparse regions 4S, the dense regions 4M and the sparse regions 4S may be disposed alternately in the extending direction.

The first threshold value Th1 may be the same value as the second threshold value Th2 (Th1 = Th2), or the second threshold value Th2 may be larger than the first threshold value Th1 (Th1 < Th2). When the second threshold value Th2 is larger than the first threshold value Th1, a slit group in which the interval between each of the plurality of slits is larger than the first threshold value Th1 and smaller than the second threshold value Th2 may be present separately from the dense slit group 5M and the sparse slit group 5S. In this case, the linear member 40 may include a region (an intermediate region) that does not correspond to either the dense region 4M or the sparse region 4S.

The intervals ΔM between each of the plurality of slits 5 in the dense slit group 5M of the linear member 40 need not necessarily be uniform, and may be different from each other within a range in which the interval ΔM satisfies the condition of being smaller than the first threshold value Th1. The intervals ΔS between each of the plurality of slits 5 in the sparse slit group 5S need not necessarily be uniform, and may be different from each other within a range in which the interval ΔS satisfies the condition of being larger than the second threshold value Th2.

The interval ΔM between each of the plurality of slits 51 in the dense slit group 5M of the linear member 41 and the interval ΔM between each of the plurality of slits 52 in the dense slit group 5M of the linear member 42 may differ from each other. The interval ΔS between each of the plurality of slits 51 in the sparse slit group 5S of the linear member 41 and the interval ΔS between each of the plurality of slits 52 in the sparse slit group 5S of the linear member 42 may differ from each other. The positions, in the extending direction, of the plurality of slits 51 of the linear member 41 and the positions, in the extending direction, of the plurality of slits 52 of the linear member 42 may be different from each other.

The boundary sections 50P in the vicinity of the apexes 400, of the boundaries between the side surfaces 402 and 403 of the linear members 40 and each of the plurality of slits 5, need not necessarily have the rounded shape, and may be angular. The pair of side surfaces 5P of each of the plurality of slits 5 may be in contact with each other. In other words, the plurality of slits 5 may be cuts formed in the linear member 40.

The hardness of the linear member 41 may be the same over the whole region in the extending direction, or the hardnesses of the dense region 41M and the sparse region 41S may be different. The hardness of the linear member 42 may be the same over the whole region in the extending direction, or the hardnesses of the dense region 42M and the sparse region 42S may be different. The hardness of the dense region 41M and the hardness of the dense region 42M may be the same as each other. The linear member 40 may be formed from a material different to the balloon 3. For example, the linear member 40 formed from the material different to the balloon 3 may adhered to the outer surface of the balloon 3 using an adhesive or the like.

The radio-opaque member 200 need not necessarily be provided at the position overlapping the dense regions 4M of the linear members 40, of the protruding section 225 of the inner tube 22. For example, the radio-opaque member 200 may be provided only at positions overlapping both ends, in the extending direction, of the dense regions 4M of the linear members 40.

The dense regions 4M of the linear members 40 may be covered by a radio-opaque material including an alloy of platinum and iridium or the like. Alternatively, the dense regions 4M of the linear members 40 may be formed using a radio-opaque material including an alloy of platinum and iridium or the like. In these cases, even if the radio-opaque member 200 is not provided at the protruding section 225 of the inner tube 22, the user can recognize the position of the dense regions 4M of the linear members 40 under X-ray illumination. On the other hand, the whole of the linear members 40 may be formed from the radio-opaque material.

### Second embodiment (Balloon catheter 1B)

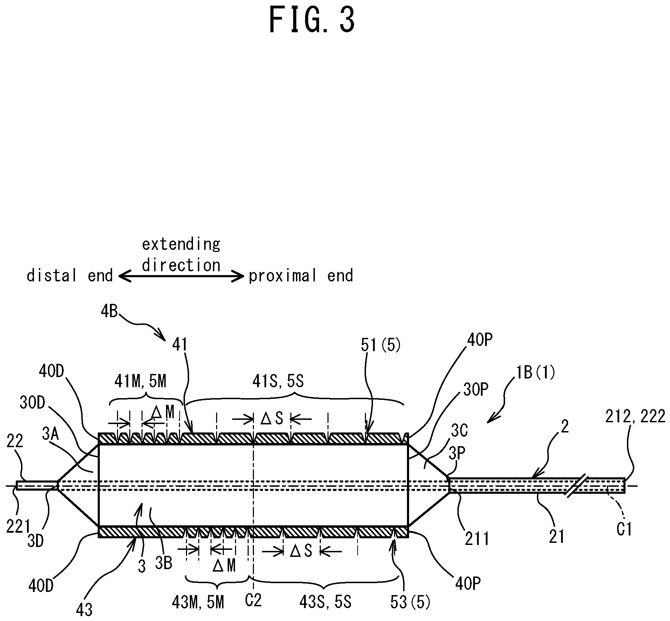

The balloon catheter 1B will be described with reference to Fig. 3. The balloon catheter 1B differs from the balloon catheter 1A (refer to Fig. 1) in that linear members 41 and 43 are provided as elements 4B. Hereinafter, the same reference signs as in Fig. 1 will be assigned to configurations that are the same as those of the balloon catheter 1A, and a description thereof will be omitted.

The linear member 41 of the elements 4B is the same as that of the balloon catheter 1A. The linear member 43 is provided in place of the linear member 42 in the balloon catheter 1A. The positions of a plurality of slits 53 formed in the linear member 43 differ from the plurality of slits 52 of the linear member 42.

In the linear member 43, a region in which the dense slit group 5M is formed, where the interval between each of the plurality of slits 53 is ΔM, will be referred to as a dense region 43M. In the linear member 43, a region in which the sparse slit group 5S is formed, where the interval between each of the plurality of slits 53 is ΔS, will be referred to as a sparse region 43S. The dense region 43M is positioned at the distal end side of the center position C2 of the linear member 43, and closer to the center position C2. The dense region 41M of the linear member 41 and the dense region 43M of the linear member 43 are disposed at different positions to each other in the extending direction. Further, both the regions 41M and 43M are disposed between the distal end portion 3D of the balloon 3 and the center position C2, more specifically, between the distal end portion 30D of the inflatable portion 3B and the center position C2 of the balloon 3. Lengths, in the extending direction, of the dense regions 41M and 43M are substantially the same as each other.

### Operations and effects of second embodiment

The dense regions 41M and 43M of the linear members 41 and 43 easily bend with the larger curvature and the sparse regions 41S and 43 S of the linear members 41 and 43 bend easily with the smaller curvature. With respect to this, in the balloon catheter 1B, by causing the positions of the dense regions 41M and 43M of the linear members 41 and 43 to be different from each other in the extending direction, it is possible to dispose the dense regions 41M and 43M at the inner side where the curvature is larger and to dispose the sparse regions 41S and 43S at the outer side where the curvature is smaller when the balloon 3 is in the bent state. As a result, the balloon catheter 1B can stably maintain the balloon 3 in the state in which the bending behavior is applied.

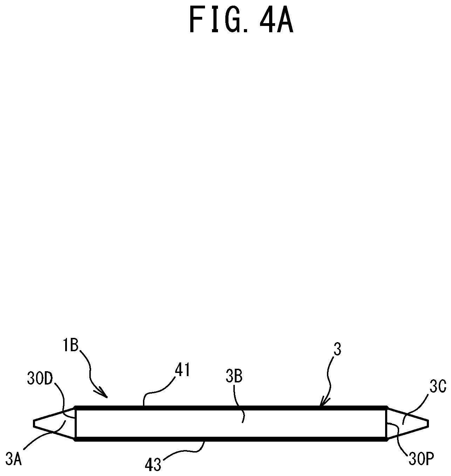

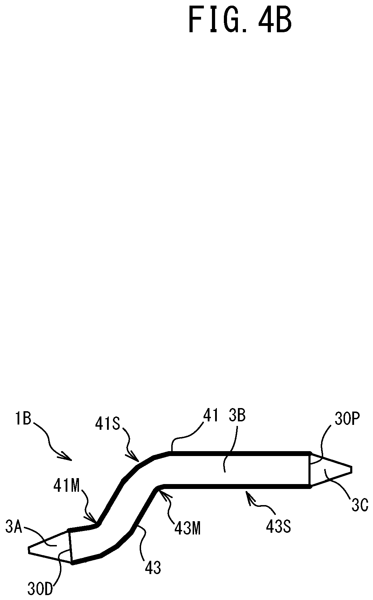

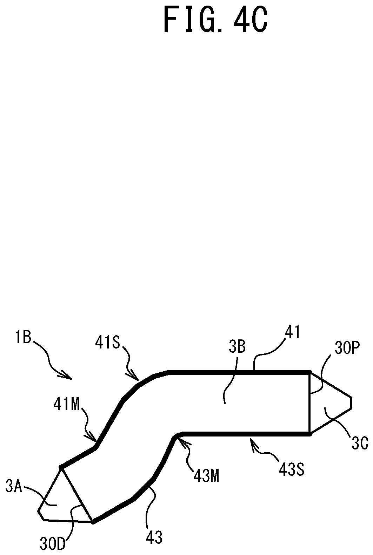

For example, the user bends the balloon 3 in the deflated state shown in Fig. 4A such that the dense region 41M of the linear member 41 and the dense region 43M of the linear member 43 are respectively disposed to the inner side. At this time, the dense region 41M of the linear member 41 and the dense region 43M of the linear member 43 curve in opposite directions. In this way, as shown in Fig. 4B, the user can form the bending behavior in the balloon 3 such that the balloon 3 is bent in two steps at positions close to the distal end portion 30D of the inflatable portion 3B. Further, the respective positions of the dense region 41M of the linear member 41 and of the dense region 43M of the linear member 43 differ from each other in the extending direction, and thus, it is possible to apply the detailed bending behavior at the positions close to the distal end portion 30D of the inflatable portion 3B. Note that the linear members 41 and 43 that have been bent while in the deflated state regulate the shape of the balloon 3 in the inflated state. Thus, as shown in Fig. 4C, the bending behavior applied to the balloon 3 in the deflated state is also maintained even after the balloon 3 is in the inflated state.

As described above, in the balloon catheter 1B, the dense regions 41M and 43M of the linear members 41 and 43 are disposed alternately so as not to overlap with each other in the extending direction. In this way, the user can easily apply, to the balloon 3, the bending behavior (an S-shaped bending behavior, for example) that is bent in multiple steps at a plurality of portions.

Furthermore, by causing the positions of the dense regions 41M and 43M of the linear members 41 and 43 to be different to each other in the extending direction, the balloon catheter 1B can distribute the force applied to the balloon 3 by the user for applying the bending behavior. Thus, the user can create the desired bending behavior of the balloon 3, without needing to make delicate adjustments in the force when the force is applied to the balloon 3.

### Special notes relating to second embodiment

The dense region 43M of the linear member 43 may be disposed straddling the distal end side and the proximal end side of the center position C2 of the balloon 3. Lengths of the dense regions 41M and 43M may be different from each other in the extending direction. A part of each of the dense regions 41M and 43M may be disposed at the same position in the extending direction. The hardnesses of the dense regions 41M and 43M may be the same as each other or may be different from each other.

In Fig. 4A to Fig. 4C, a case is exemplified in which the balloon 3 is bent such that both the dense region 41M of the linear member 41 and the dense region 43M of the linear member 43 are curved, but the configuration is not limited to this example. For example, the balloon 3 may be bent such that only one of the dense region 41M of the linear member 41 and the dense region 43M of the linear member 43 is curved.

### Third embodiment (Balloon catheter 1C)

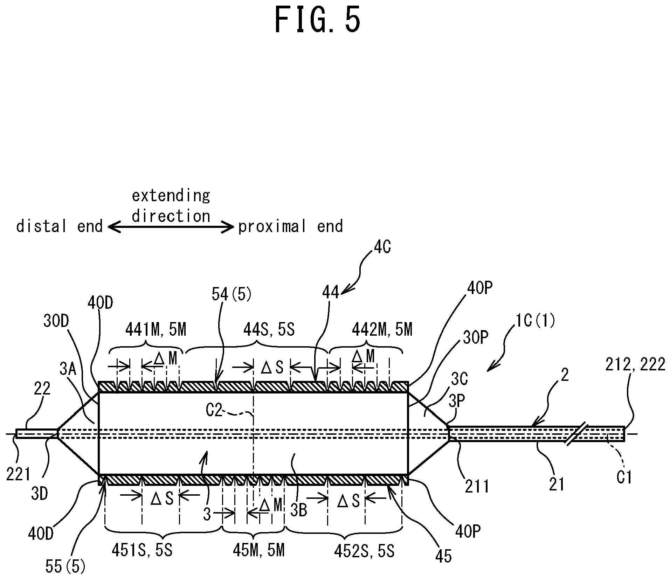

The balloon catheter 1C will be described with reference to Fig. 5. The balloon catheter 1C differs from the balloon catheter 1A (refer to Fig. 1) in that linear members 44 and 45 are provided as elements 4C. Hereinafter, the same reference signs as in Fig. 1 will be assigned to configurations that are the same as those of the balloon catheter 1A, and a description thereof will be omitted.

In the linear member 44, regions in which the dense slit groups 5M are formed, where the interval between each of a plurality of slits 54 is ΔM, will be referred to as a dense region 441M and a dense region 442M. In the linear member 44, a region in which the sparse slit group 5S is formed, where the interval between each of the plurality of slits 54 is ΔS, will be referred to as a sparse region 44S.

Of the linear member 44, the dense region 441M is disposed close to the distal end portion 30D of the inflatable portion 3B of the balloon 3. Of the linear member 44, the dense region 442M is disposed close to the proximal end portion 30P of the inflatable portion 3B of the balloon 3. Of the linear member 44, the sparse region 44S is disposed to the proximal end side of the dense region 441M and to the distal end side of the dense region 442M. The sparse region 44S is sandwiched from both sides by the dense regions 441M and 442M in the extending direction. The sparse region 44S is disposed straddling the distal end side and the proximal end side of the center position C2 of the balloon 3. In other words, the sparse region 44S of the linear member 44 includes the center position C2.

In the linear member 45, a region in which the dense slit group 5M is formed, where the interval between each of a plurality of slits 55 is ΔM, will be referred to as a dense region 45M. In the linear member 45, regions in which the sparse slit groups 5S are formed, where the interval between each of the plurality of slits 55 is ΔS, will be referred to as a sparse region 451S and a sparse region 452S.

Of the linear member 45, the sparse region 451S is disposed close to the distal end portion 30D of the inflatable portion 3B of the balloon 3. Of the linear member 45, the sparse region 452S is disposed close to the proximal end portion 30P of the inflatable portion 3B of the balloon 3. Of the linear member 45, the dense region 45M is disposed to the proximal end side of the sparse region 451S and to the distal end side of the sparse region 452S. The dense region 45M is sandwiched from both sides by the sparse regions 451S and 452S in the extending direction. The dense region 45M is disposed straddling the distal end side and the proximal end side of the center position C2 of the balloon 3. In other words, the dense region 45M of the linear member 45 includes the center position C2.

The end portion on the proximal end side of the dense region 441M of the linear member 44 is positioned further to the distal end side than the end portion on the distal end side of the dense region 45M of the linear member 45. The dense regions 441M and 45M do not overlap each other in the extending direction. The end portion on the distal end side of the dense region 442M of the linear member 44 is positioned further to the proximal end side than the end portion on the proximal end side of the dense region 45M of the linear member 45. The dense regions 442M and 45M do not overlap each other in the extending direction. In other words, the dense regions 441M, 442M, and 45M are disposed at different positions in the extending direction. Further, the dense region 45M is disposed between the dense regions 441M and 442M in the extending direction.

### Operations and effects of third embodiment

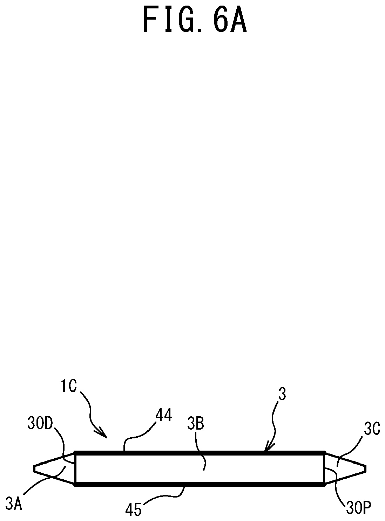

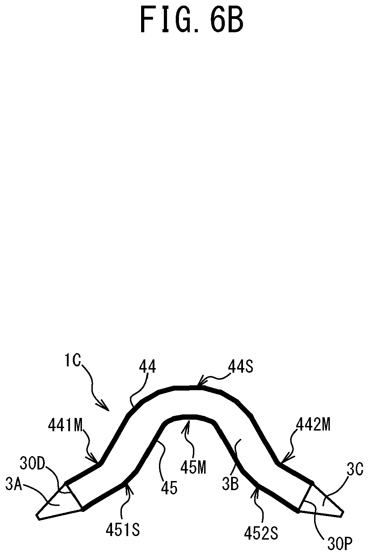

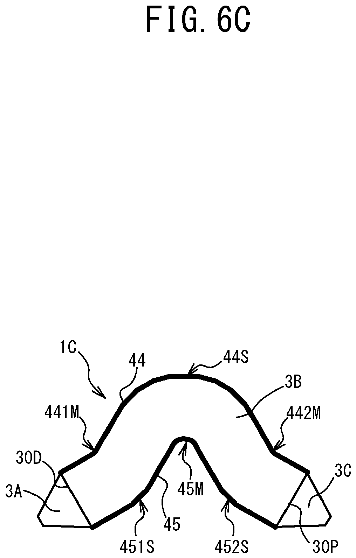

For example, the user bends the balloon 3 in the deflated state shown in Fig. 6A such that the dense regions 441M, 45M, and 441M are respectively disposed to the inner side. At this time, the dense regions 441M and 45M curve in opposite directions, and the dense regions 45M and 442M curve in opposite directions. In this way, as shown in Fig. 6B, the user can apply the bending behavior to the balloon 3 such that the balloon 3 is alternately bent in directions intersecting the extending direction. Note that the linear members 44 and 45 that have been bent while in the deflated state regulate the shape of the balloon 3 in the inflated state. Thus, as shown in Fig. 6C, the bending behavior applied to the balloon 3 in the deflated state is also maintained even after the balloon 3 is in the inflated state.

As described above, in the balloon catheter 1C, the dense regions 441M, 442M, and 45M of the linear members 44 and 45 are disposed at the different positions, and the dense region 45M is disposed between the dense regions 441M and 442M. In this way, the user can easily apply, to the balloon 3, the bending behavior that is bent in multiple steps at a greater number of portions than in the balloon catheter 1B.

In the balloon catheter 1C, the dense region 45M of the linear member 45 includes the center position C2. Thus, the user can easily apply the bending behavior such that the curvature at the center position C2 in the extending direction of the balloon 3 becomes larger.

### Special notes relating to third embodiment

The dense regions 441M, 45M, and 442M may all be disposed further to the distal end side than the center position C2. Alternatively, the dense regions 441M and 45M may be disposed further to the distal end side than the center position C2, and the dense region 442M may be disposed further to the proximal end side than the center position C2. The hardnesses of the dense regions 441M, 45M, and 442M may be the same as each other or may differ from each other. For example, the hardnesses of the dense regions 441M and 442M may be the same as each other and may each be different from the hardness of the dense region 45M.

### Fourth embodiment (Balloon catheter 1D)

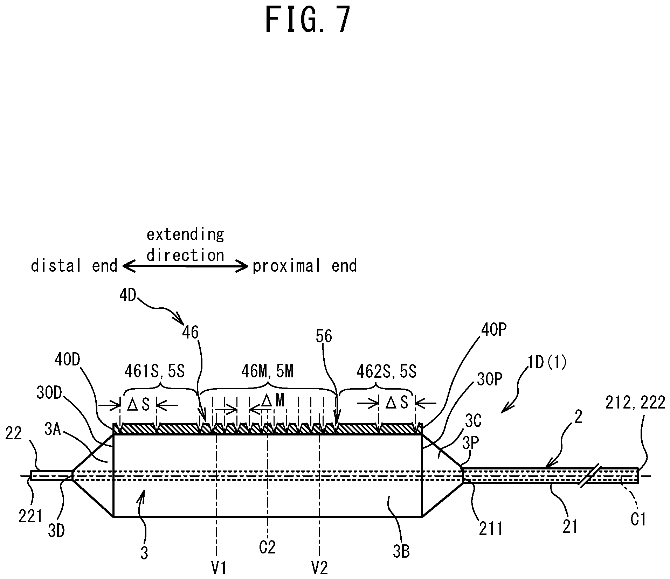

The balloon catheter 1D will be described with reference to Fig. 7. The balloon catheter 1D differs from the balloon catheter 1A (refer to Fig. 1) in that only a linear member 46 is provided as an element 4D. Hereinafter, the same reference signs as in Fig. 1 will be assigned to configurations that are the same as those of the balloon catheter 1A, and a description thereof will be omitted.

In the linear member 46, a region in which the dense slit group 5M is formed, where the interval between each of a plurality of slits 56 is ΔM, will be referred to as a dense region 46M. In the linear member 46, regions in which the sparse slit groups 5S are formed, where the interval between each of the plurality of slits 56 is ΔS, will be referred to as a sparse region 461S and a sparse region 462S. Of the linear member 46, the sparse region 461S is disposed close to the distal end portion 30D of the inflatable portion 3B of the balloon 3. Of the linear member 46, the sparse region 462S is disposed close to the proximal end portion 30P of the inflatable portion 3B of the balloon 3. Of the linear member 46, the dense region 46M is disposed to the proximal end side of the sparse region 461S and to the distal end side of the sparse region 462S. The dense region 46M is sandwiched from both sides by the sparse regions 461S and 462S in the extending direction. The dense region 46M is disposed straddling the distal end side and the proximal end side of the center position C2. In other words, the dense region 46M of the linear member 46 includes the center position C2.

Equal division positions V1 and V2 that divide the inflatable portion 3B of the balloon 3 into thirds are defined. The end portion on the distal end side of the dense region 46M is disposed to the distal end side of the equal division position V1 that is positioned on the distal end side than the equal division position V2. The end portion on the proximal end side of the dense region 46M is disposed to the proximal end side of the equal division position V2 that is positioned on the proximal end side than the equal division position V1. In other words, the dense region 46M of the linear member 46 includes the equal division positions V1 and V2. The length of the dense region 46M in the extending direction is longer than the length of the third of the inflatable portion 3B of the balloon 3.

### Operations and effects of fourth embodiment

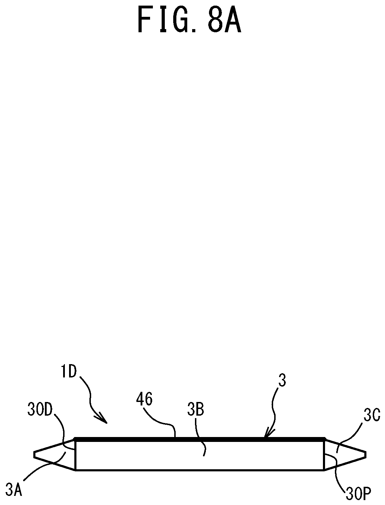

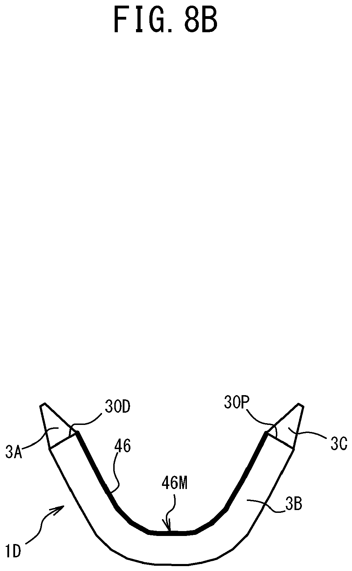

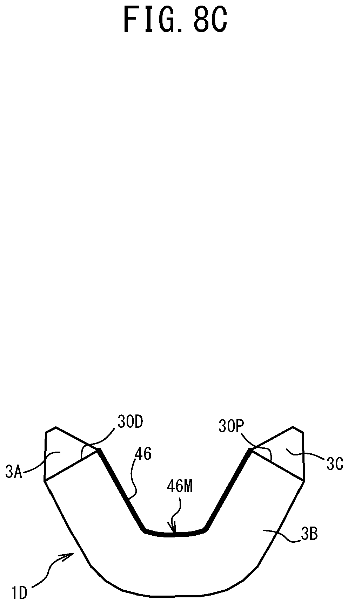

For example, the user bends the balloon 3 in the deflated state shown in Fig. 8A such that the dense region 46M of the linear member 46 is disposed to the inner side. In this way, as shown in Fig. 8B, the user can form the bending behavior in the balloon 3 such that the curvature of a portion corresponding to the center position C2 of the balloon 3 is larger. Note that the linear member 46 that has been bent while in the deflated state regulate the shape of the balloon 3 in the inflated state. Thus, as shown in Fig. 8C, the bending behavior applied to the balloon 3 in the deflated state is also maintained even after the balloon 3 is in the inflated state.

As described above, the dense region 46M of the balloon catheter 1D is disposed at a position including the center position C2. In this way, the user can easily apply, to the balloon 3, the bending behavior (an L shape or a U shape, for example) that is a shape where the balloon 3 is bent at the center position C2.

The dense region 46M of the linear member 46 includes the equal division positions V1 and V2 that divide the inflatable portion 3B of the balloon 3 into thirds. In other words, of the linear member 46, the dense region 46M occupies a wide region including the center position C2 of the balloon 3. Thus, when the user applies a bending behavior at which the curvature at the center position C2 of the balloon 3 is large, it is possible to suppress a force from acting locally on the balloon 3 and to suppress the balloon 3 from being bent at an acute angle.

### Special notes relating to fourth embodiment

The length of the dense region 46M of the linear member 46 in the extending direction is not limited to that of the above-described embodiment. For example, the dense region 46M of the linear member 46 may include three equal division positions that divide the inflatable portion 3B of the balloon 3 into quarters. In this case, the balloon catheter 1D can more effectively suppress the balloon 3 from being bent at an acute angle when the user applies the bending behavior to the balloon 3.

The balloon catheter 1D may include another linear member other than the linear member 46, as the element 4D. For example, the other linear member may extend in the extending direction along the outer surface of the inflatable portion 3B, and may be disposed at a position facing the linear member 46 with the center axis C1 interposed therebetween. Furthermore, the other linear member may include the plurality of slits 56 of the sparse slit group only. In other words, the whole of the other linear member may be the sparse region. Further, a dense region may be formed in the other linear member at a position that is the same, in the extending direction, as the position of the dense region 46M of the linear member 46. In this case, the balloon catheter 1D can hold the bending behavior of the balloon 3 using the plurality of linear members, and can thus stably maintain the balloon 3 in the state in which the bending behavior is applied.

### Fifth embodiment (Balloon catheter 1E)

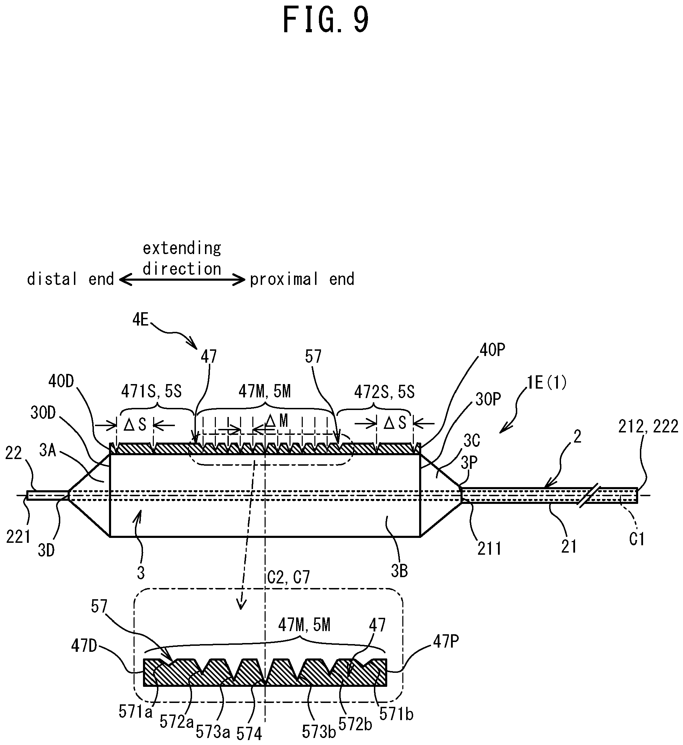

The balloon catheter 1E will be described with reference to Fig. 9. The balloon catheter 1E differs from the balloon catheter 1D (refer to Fig. 7) in that a linear member 47 is provided as an element 4E. Hereinafter, the same reference signs as in Fig. 7 will be assigned to configurations that are the same as those of the balloon catheter 1D, and a description thereof will be omitted.

The linear member 47 includes a dense region 47M and sparse regions 471S and 472S. The dense region 47M and the sparse regions 471S and 472S are respectively disposed at the same positions as the dense region 46M and the sparse regions 461S and 462S of the linear member 46 (refer to Fig. 7). The hardness of the linear member 47 is uniform over the whole region in the extending direction. In the linear member 47, the greatest depth of each of a plurality of slits 57 in the dense slit group 5M of the dense region 47M is different to that in the linear member 46.

Slits 571a, 571b, 572a, 572b, 573a, 573b, and 574 are included in the dense slit group 5M of the dense region 47M, as the plurality of slits 57. The slits 571a, 572a, 573a, 574, 573b, 572b, and 571b are arranged, in that order, from a distal end portion 47D that is the end portion on the distal end side of the dense region 47M, to a proximal end portion 47P that is the end portion on the proximal end side of the dense region 47M.

The interval between the distal end portion 47D and the slit 571a, and the interval between the proximal end portion 47P and the slit 571b are the same as each other. The interval between the distal end portion 47D and the slit 572a, and the interval between the proximal end portion 47P and the slit 572b are the same as each other. The interval between the distal end portion 47D and the slit 573a, and the interval between the proximal end portion 47P and the slit 573b are the same as each other. The slit 574 is disposed at a center position C7 between the distal end portion 47D and the proximal end portion 47P. The center position C7 is aligned with the center position C2 of the balloon 3. The greatest width of each of the slits 571a, 571b, 572a, 572b, 573a, 573b, and 574 is the same.

The greatest depth of the slits 571a and 571b is the same. Hereinafter, this greatest depth will be denoted by d1. The greatest depth of the slits 572a and 572b is the same. Hereinafter, this greatest depth will be denoted by d2. The greatest depth of the slits 573a and 573b is the same. Hereinafter, this greatest depth will be denoted by d3. The greatest depth of the slit 574 will be denoted by d4. In this case, the greatest depths d1 to d4 have the following relationship: d1 < d2 < d3 < d4. In other words, the greatest depths of each of the slits 571a, 571b, 572a, 572b, 573a, 573b, and 574 of the dense slit group 5M become gradually deeper the further toward the center position C7 from the distal end portion 47D and the proximal end portion 47P of the dense region 47M of the linear member 47.

### Operations and effects of fifth embodiment

In the linear member 47, the deeper the greatest depth of each of the plurality of slits 57, the more it is possible to curve with the larger curvature. In other words, the dense region 47M of the linear member 47 can curve with the larger curvature the closer to the center position C7. Note that, when the linear member 47 curves at the dense region 47M, the closer to the center position C7 of the dense region 47M, the more easily the larger curvature is obtained. Thus, the user can easily form the state in which the dense region 47M of the linear member 47 is curved, and can apply the bending behavior to the balloon 3.

### Special notes relating to fifth embodiment

The position of the dense region 47M in the linear member 47 is not limited to the position including the center position C2. For example, the dense region 47M may be disposed further to the distal end side or the proximal end side than the center position C2. The linear member 47 may include a plurality of dense regions. Then, for each of the plurality of dense regions, the greatest depth of each of the plurality of slits 57 in the dense slit group may become deeper the further toward a center position of the dense region.

The greatest depths of the slits 571a and 571b may be different from each other. The greatest depths of the slits 572a and 572b may be different from each other. The greatest depths of the slits 573a and 573b may be different from each other. The greatest depth of each of the slits 571a, 571b, 572a, 572b, 573a, 573b, and 574 may become gradually deeper the closer to a position displaced from the center position C7 to the distal end side or the proximal end side.

The greatest depth of each of the plurality of slits 57 in the dense slit group 5M of the dense region 47M may have a correlation with a distance to the distal end portion 47D or the proximal end portion 47P of the dense region 47M. For example, of the dense slit group 5M of the dense region 47M, for the plurality of slits 57 disposed further to the distal end side than the center position C7, each of the greatest depths may be prescribed such that a value obtained by multiplying the distance to the distal end portion 47D by the greatest depth is constant. Further, of the dense slit group 5M of the dense region 47M, for the plurality of slits 57 disposed further to the proximal end side than the center position C7, each of the greatest depths may be prescribed such that a value obtained by multiplying the distance to the proximal end portion 47Pby the greatest depth is constant.

The intervals between each of the plurality of slits 57 in the dense slit group 5M of the dense region 47M may be different from each other. For example, the interval between each of the plurality of slits 57 in the dense slit group 5M of the dense region 47M may become gradually smaller or may become gradually larger toward the center position C7 from the distal end portion 47D or the proximal end portion 47P of the dense region 47M.

### Sixth embodiment (Balloon catheter 1F)

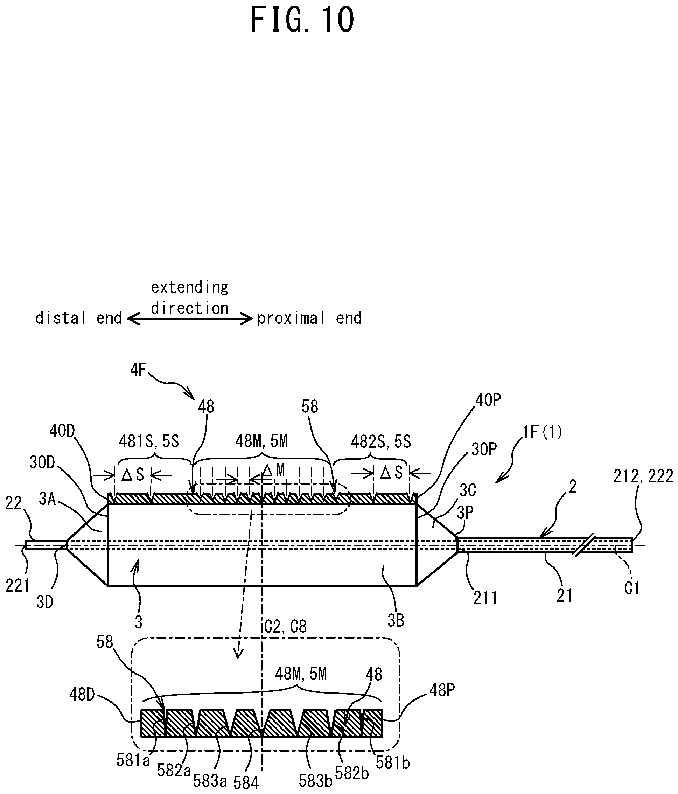

The balloon catheter 1F will be described with reference to Fig. 10. The balloon catheter 1F differs from the balloon catheter 1D (refer to Fig. 7) and the balloon catheter 1E (refer to Fig. 9) in that a linear member 48 is provided as an element 4F. Hereinafter, the same reference signs as in Fig. 7 and Fig. 9 will be assigned to configurations that are the same as those of the balloon catheters 1D and 1E, and a description thereof will be omitted.

The linear member 48 includes a dense region 48M and sparse regions 481S and 482S. The dense region 48M and the sparse regions 481S and 482S are respectively disposed at the same positions as the dense region 47M and the sparse regions 471S and 472S of the linear member 47 (refer to Fig. 9). The hardness of the linear member 48 is uniform over the whole region in the extending direction. In the linear member 48, the greatest width of each of a plurality of slits 58 in the dense slit group 5M of the dense region 48M is different to that in the linear member 47.

Slits 581a, 581b, 582a, 582b, 583a, 583b, and 584 are included in the dense slit group 5M, as the plurality of slits 58. The slits 581a, 582a, 583a, 584, 583b, 582b, and 581b are arranged, in that order, from a distal end portion 48D that is the end portion on the distal end side of the dense region 48M, to a proximal end portion 48P that is the end portion on the proximal end side of the dense region 48M.

The distance between the distal end portion 48D and the slit 581a, and the distance between the proximal end portion 48P and the slit 581b are the same as each other. The distance between the distal end portion 48D and the slit 582a, and the distance between the proximal end portion 48P and the slit 582b are the same as each other. The distance between the distal end portion 48D and the slit 583a, and the distance between the proximal end portion 48P and the slit 583b are the same as each other. The slit 584 is disposed at a center position C8 between the distal end portion 48D and the proximal end portion 48P. The center position C8 is aligned with the center position C2 of the inflatable portion 3B of the balloon 3. The greatest depth of each of the slits 581a, 581b, 582a, 582b, 583a, 583b, and 584 is the same.

The greatest width of the slits 581a and 581b is the same. Hereinafter, this greatest width will be denoted by w1. The greatest width of the slits 582a and 582b is the same. Hereinafter, this greatest width will be denoted by w2. The greatest width of the slits 583a and 583b is the same. Hereinafter, this greatest width will be denoted by w3. The greatest width of the slit 584 will be denoted by w4. In this case, the greatest widths w1 to w4 have the following relationship: w1 < w2 < w3 < w4. In other words, the greatest widths of each of the slits 581a, 581b, 582a, 582b, 583a, 583b, and 584 included in the dense slit group 5M become gradually larger the further toward the center position C8 from the distal end portion 48D and the proximal end portion 48P of the dense region 48M of the linear member 48.

### Operations and effects of sixth embodiment

In the linear member 48, the wider the maximum width of each of the plurality of slits 58, the more it is possible to bend with the larger curvature. In other words, the dense region 48M of the linear member 48 can curve with the larger curvature the closer to the center position C8. Note that, when the linear member 48 curves at the dense region 48M, the closer to the center position C8 of the dense region 48M, the more easily the larger curvature is obtained. Thus, the user can easily form the state in which the dense region 48M of the linear member 48 is curved, and can apply the bending behavior to the balloon 3.

### Special notes relating to sixth embodiment

The position of the dense region 48M in the linear member 48 is not limited to the position including the center position C2. For example, the dense region 48M may be disposed further to the distal end side or the proximal end side than the center position C2. The linear member 48 may include a plurality of dense regions. Then, for each of the plurality of dense regions, the greatest width of each of the plurality of slits 58 in the dense slit group may become larger the further toward a center position of the dense region.

The greatest widths of the slits 581a and 581b may be different from each other. The greatest widths of the slits 582a and 582b may be different from each other. The greatest widths of the slits 583a and 583b may be different from each other. The greatest width of each of the slits 581a, 581b, 582a, 582b, 583a, 583b, and 584 may become gradually larger the closer to a position displaced from the center position C8 to the distal end side or the proximal end side.

The greatest width of each of the plurality of slits 58 in the dense slit group 5M of the dense region 48M may have a correlation with a distance to the distal end portion 48D or the proximal end portion 48P of the dense region 48M. For example, of the dense slit group 5M of the dense region 48M, for the plurality of slits 58 disposed further to the distal end side than the center position C8, each of the greatest widths may be prescribed such that a value obtained by multiplying the distance to the distal end portion 48D by the greatest width is constant. Further, of the dense slit group 5M of the dense region 48M, for the plurality of slits 58 disposed further to the proximal end side than the center position C8, each of the greatest widths may be prescribed such that a value obtained by multiplying the distance to the proximal end portion 48Pby the greatest width is constant.

The intervals between each of the plurality of slits 58 in the dense slit group 5M of the dense region 48M may be different from each other. For example, the interval between each of the plurality of slits 58 in the dense slit group 5M of the dense region 48M may become gradually smaller or may become gradually larger toward the center position C8 from the distal end portion 48D or the proximal end portion 48P of the dense region 48M in the linear member 48.

The greatest depths of the slits 581a, 581b, 582a, 582b, 583a, 583b, and 584 need not necessarily be the same as each other. For example, in a similar manner to the linear member 47 of the balloon catheter 1E, the greatest depth of each of the plurality of slits 58 in the dense region 48M of the linear member 48 may become gradually deeper toward the center position C8 from the distal end portion 48D and the proximal end portion 48P.

### Seventh embodiment (Balloon catheter 1G)

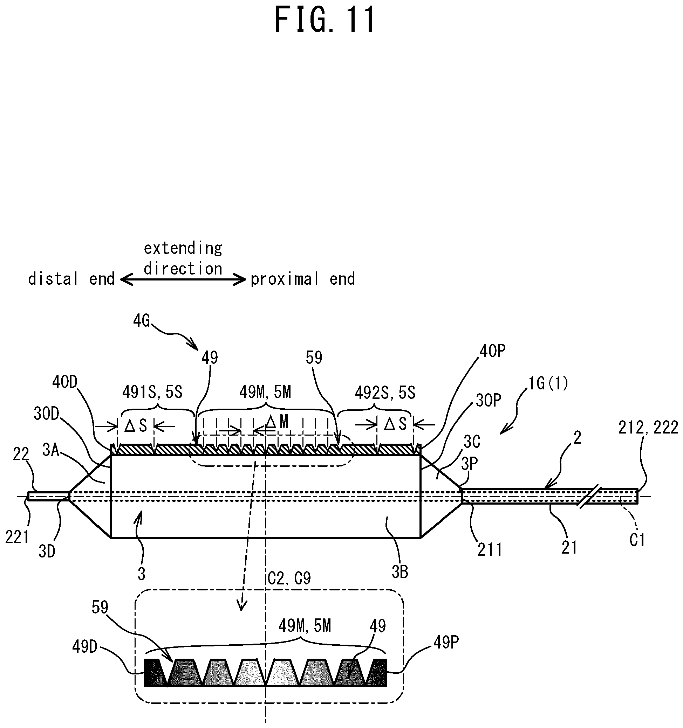

The balloon catheter 1G will be described with reference to Fig. 11. The balloon catheter 1G differs from the balloon catheter 1D (refer to Fig. 7), the balloon catheter 1E (refer to Fig. 9), and the balloon catheter 1F (refer to Fig. 10) in that a linear member 49 is provided as an element 4G. Hereinafter, the same reference signs as in Fig. 7, Fig. 9, and Fig. 10 will be assigned to configurations that are the same as those of the balloon catheters 1D to 1F and a description thereof will be omitted.

The linear member 49 includes a dense region 49M and sparse regions 491S and 492S. The dense region 49M and the sparse regions 491S and 492S are respectively disposed at the same positions as the dense region 47M and the sparse regions 471S and 472S of the linear member 47 (refer to Fig. 9), or as the dense region 48M and the sparse regions 481S and 482S of the linear member 48 (refer to Fig. 10).

In the linear member 49, the hardness of the dense region 49M differs from that of the linear members 47 and 48, and is not uniform. Specifically, the hardness of the dense region 49M of the linear member 49 is hardest at a distal end portion 49D that is the end portion on the distal end side of the dense region 49M and at a proximal end portion 49P that is the end portion on the proximal end side of the dense region 49M. On the other hand, the hardness of the dense region 49M of the linear member 49 is softest at a center position C9 between the distal end portion 498D and the proximal end portion 48P. In other words, the hardness of the dense region 49M of the linear member 49 becomes gradually softer toward the center position C9 from the distal end portion 49D and the proximal end portion 49P of the dense region 49M. Note that the center position C9 is aligned with the center position C2 of the inflatable portion 3B of the balloon 3.

### Operations and effects of seventh embodiment

In the linear member 49, the softer the hardness, the more easily the linear member 49 bends and tracks changes in curvature in the blood vessel. Here, since the dense region 49M of the linear member 49 becomes softer toward the center position C9, the closer to the center position C9, the more easily the linear member 49 bends. Further, when the linear member 49 bends at the dense region 49M, the curvature becomes larger more easily the closer to the center position C9 of the dense region 49M. Thus, the balloon catheter 1G can easily form the linear member 49 to be in the bent state when the balloon 3 bends to track the blood vessel.

Further, the user can form the bending behavior in the balloon 3 by easily forming the state in which the dense region 49M of the linear member 49 is bent.

### Special notes relating to seventh embodiment

At extent of change when the hardness of the dense region 49M of the linear member 49 changes toward the center position C9 may be linear or may be non-linear. For example, the extent of change of the hardness of the dense region 49M may become larger the closer to the center position C9. The hardness of the dense region 49M of the linear member 49 may become continuously softer toward the center position C9 from the distal end portion 49D and the proximal end portion 49P, or may become softer in a step-wise manner. The hardness of the linear member 49 may become gradually softer toward a position displaced from the center position C9 to the distal end side or the proximal end side.

The greatest depth may be the same for each of the plurality of slits 59 in the dense slit group 5M of the dense region 49M, or may become gradually deeper from the distal end portion 49D and the proximal end portion 49P toward the center position C9. The greatest width may be the same for each of the plurality of slits 59 in the dense slit group 5M of the dense region 49M, or may become gradually larger from the distal end portion 49D and the proximal end portion 49P toward the center position C9.

### Other

The present invention is not limited to the above-described embodiments, and various modifications are possible. The linear members 41 to 49 described above to exemplify the balloon catheters 1A to 1G can be combined with each other as appropriate. The special notes described for each of the first to seventh embodiments can be applied to the other embodiments as appropriate.

The distal end portion 3D of the balloon 3 is an example of a first distal end portion of the present invention. The proximal end portion 3P of the balloon 3 is an example of a first proximal end portion of the present invention. The distal end portion 40D is an example of a second distal end portion of the present invention. The proximal end portion 40P is an example of a second proximal end portion of the present invention. The distal end 221 is an example of a third distal end portion of the present invention. The proximal ends 212 and 222 are an example of a third proximal end portion of the present invention.

## Claims

1. A balloon for a catheter comprising:
a balloon extending between a first distal end portion being an end portion on one side in an extending direction and a first proximal end portion being an end portion on another side in the extending direction; and
an element disposed at at least a part of an outer surface of the balloon, the element including at least one linear member extending in the extending direction between a second distal end portion being an end portion close to the first distal end portion and a second proximal end portion being an end portion close to the first proximal end portion, wherein
the linear member includes a plurality of slits arranged in the extending direction, and
the linear member includes a dense region where an interval between each of the plurality of slits is smaller than a predetermined first threshold value, and a sparse region where the interval between each of the plurality of slits is larger than a second threshold value being a value equal to or greater than the first threshold value.

2. The balloon for a catheter according to claim 1, wherein
the element includes a plurality of the linear members, and
the plurality of linear members is arranged in a circumferential direction centered on a center axis of the balloon.

3. The balloon for a catheter according to claim 2, wherein
the plurality of linear members includes at least a first linear member and a second linear member, and
the dense region of the first linear member and the dense region of the second linear member are disposed at different positions from each other in the extending direction.

4. The balloon for a catheter according to claim 3, wherein
the first linear member includes a first dense region and a second dense region as the dense region,
the second linear member includes a third dense region as the dense region,
the first dense region, the second dense region, and the third dense region are disposed at different positions from each other in the extending direction, and
in the extending direction, the third dense region is disposed between the first dense region and the second dense region.

5. The balloon for a catheter according to claim 3, wherein
the first linear member includes a first dense region as the dense region,
the second linear member includes a second dense region as the dense region,
the first dense region and the second dense region are disposed at different positions from each other in the extending direction, and
in the extending direction, the first dense region and the second dense region are disposed between the first distal end portion and a center position between the first distal end portion and the first proximal end portion of the balloon.

6. The balloon for a catheter according to claim 2, wherein
the plurality of linear members includes at least a first linear member and a second linear member,
the first linear member includes a first dense region as the dense region,
the second linear member includes a second dense region as the dense region,
the first dense region and the second dense region are disposed at the same position as each other in the extending direction, and
in the extending direction, the first dense region and the second dense region are disposed between the first distal end portion and a center position between the first distal end portion and the first proximal end portion of the balloon.

7. The balloon for a catheter according to any one of claims 2 to 6, wherein
the plurality of linear members includes at least a first linear member and a second linear member, and
a hardness at the dense region of the first linear member is different from a hardness at the dense region of the second linear member.

8. The balloon for a catheter according to any one of claims 1 to 4, wherein
in the extending direction, a position at which the dense region of the linear member is disposed includes a center position in the extending direction between the first distal end portion and the first proximal end portion of the balloon.

9. The balloon for a catheter according to claim 8, wherein
the balloon includes
an inflatable portion having a tube shape and extending in the extending direction,
a distal end connecting portion being a portion extending to the first distal end portion from an end portion on the one side in the extending direction of the inflatable portion, a diameter of an end portion connected to the inflatable portion being larger than a diameter of the first distal end portion, and
a proximal end connecting portion being a portion extending to the first proximal end portion from an end portion on the other side in the extending direction of the inflatable portion, a diameter of an end portion connected to the inflatable portion being larger than a diameter of the first proximal end portion, and
in the extending direction, a position at which the dense region of the linear member is disposed includes the center position, and includes two equal division positions dividing the inflatable portion into thirds in the extending direction.

10. The balloon for a catheter according to any one of claims 1 to 9, wherein
a greatest depth, in a radial direction centered on a center axis of the balloon, of each of the plurality of slits becomes gradually deeper toward a central portion of the dense region from both end portions thereof in the extending direction.

11. The balloon for a catheter according to any one of claims 1 to 10, wherein
each of the plurality of slits is formed by a pair of side surfaces facing each other in the extending direction, and
a greatest width, in the extending direction, between the pair of side surfaces of each of the plurality of slits becomes gradually larger toward a central portion of the dense region from both end portions thereof in the extending direction.

12. The balloon for a catheter according to any one of claims 1 to 11, wherein
a hardness of the linear member becomes gradually softer toward a central portion of the dense region from both end portions thereof in the extending direction.

13. The balloon for a catheter according to any one of claims 1 to 12, wherein
the dense region of the linear member is covered by a radio-opaque material.

14. The balloon for a catheter according to any one of claims 1 to 13, wherein
at least the dense region of the linear member is formed from a radio-opaque material.

15. The balloon for a catheter according to any one of claims 1 to 14, wherein
a boundary section between an outer surface of the linear member and the slit has a rounded shape.

16. The balloon for a catheter according to any one of claims 1 to 15, wherein
the balloon and the element are formed from the same material.

17. A balloon catheter comprising:
the balloon for a catheter according to any one of claims 1 to 16; and
a shaft extending between a third distal end portion being an end portion on one side and a third proximal end portion being an end portion on another side, wherein
the first distal end portion of the balloon for a catheter is disposed close to the third distal end portion of the shaft, and the first proximal end portion of the balloon for a catheter is disposed at a position separated from the third distal end portion of the shaft to the third proximal end portion side, and
the shaft includes a radio-opaque material indicating a position of the dense region.
